(19) **Europäisches Patentamt**
**European Patent Office**
**Office européen des brevets**

(11) **EP 1 874 945 B1**

(12) **EUROPÄISCHE PATENTSCHRIFT**

(45) Veröffentlichungstag und Bekanntmachung des Hinweises auf die Patenterteilung:
**01.09.2010 Patentblatt 2010/35**

(21) Anmeldenummer: **06742583.5**

(22) Anmeldetag: **13.04.2006**

(51) Int Cl.:
*C12P 13/04* (2006.01)   *C07C 227/40* (2006.01)
*C07C 229/02* (2006.01)

(86) Internationale Anmeldenummer:
**PCT/EP2006/003431**

(87) Internationale Veröffentlichungsnummer:
**WO 2006/108663 (19.10.2006 Gazette 2006/42)**

(54) **VERFAHREN ZUR GEWINNUNG EINER BASISCHEN AMINOSÄURE AUS EINER FERMENTATIONSBRÜHE**

METHOD FOR RECOVERING A BASIC AMINO ACID FROM A FERMENTATION LIQUOR

PROCEDE POUR EXTRAIRE UN ACIDE AMINE BASIQUE D'UN BOUILLON DE FERMENTATION

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **15.04.2005 DE 102005017507**

(43) Veröffentlichungstag der Anmeldung:
**09.01.2008 Patentblatt 2008/02**

(73) Patentinhaber: **Paik Kwang Industrial Co., Ltd.**
**Jeollabuk-do (KR)**

(72) Erfinder:
 • **HAN, Duck-Keun**
**Gunsan, Jeonbuk (KR)**
 • **CHOI, Jong-Kyu**
**201-1103 Seoho**
**Jeonju,**
**Jeonbuk (KR)**
 • **HONG, Il-Kwon**
**Gunsan, Jeonbuk (KR)**
 • **KIM, Hyun-Ho**
**Gunsan Jeonbuk (KR)**
 • **CHOI, Jong-Soo**
**Gangseo-Gu, Seoul (KR)**
 • **KIM, Tae-Hui**
**Gunsan, Jeonbuk (KR)**
 • **KIM, Sung Hyun**
**Gunsan, Jeonbuk (KR)**

(74) Vertreter: **Maiwald Patentanwalts GmbH**
**Elisenhof**
**Elisenstrasse 3**
**80335 München (DE)**

(56) Entgegenhaltungen:
**EP-A- 1 106 602**

 • **PATENT ABSTRACTS OF JAPAN Bd. 012, Nr. 139 (C-491), 27. April 1988 (1988-04-27) & JP 62 255452 A (AJINOMOTO CO INC), 7. November 1987 (1987-11-07)**
 • **PATENT ABSTRACTS OF JAPAN Bd. 012, Nr. 139 (C-491), 27. April 1988 (1988-04-27) & JP 62 255453 A (AJINOMOTO CO INC), 7. November 1987 (1987-11-07)**

## Beschreibung

Die vorliegende Erfindung betrifft ein Verfahren zur Gewinnung einer basischen Aminosäure aus der Fermentationsbrühe eines die basische Aminosäure produzierenden Mikroorganismenstamms.

Basische Aminosäuren wie L-Lysin, L-Histidin, L-Arginin und L-Ornithin werden überwiegend durch mikrobielle Fermentationsverfahren hergestellt (s. z.B. Axel Kleemann et al., "Amino acids", in "Ullmann's Encyclopedia of Industrial Chemistry", 5th Edition on CD-ROM, 1997 Wiley-VCH und dort zitierte Literatur; Th. Hermann, J. Biotechnol. 104 (2003), S. 155 - 172 und dort zitierte Literatur; Pfefferle et al., Adv. Biochem. Eng./Biotechnology, Vol. 79 (2003), 59 -112 und dort zitierte Literatur; sowie Atkinson et al., in Biochemical Engineering and Biotechnology Handbook, 2nd ed., Stockton Press, 1991, Kapitel 20 und dort zitierte Literatur).

Bei derartigen Fermentationsverfahren erhält man primär eine wässrige Fermentationsbrühe, welche neben der gewünschten basischen Aminosäure und der aus den eingesetzten Mikroorganismen resultierenden Biomasse eine Vielzahl an Nebenprodukten und Verunreinigungen, z.B. andere Aminosäuren, Substratreste, Salze, Produkte der Zelllyse, und sonstige Nebenprodukte enthält.

Die Gewinnung basischer Aminosäuren aus der Fermentationsbrühe und ihre Aufreinigung erfolgt häufig unter Anwendung von stark sauren Kationentauschern (s. z.B. Th. Hermann, loc.cit; Atkinson et al., loc. cit.). Zu diesem Zweck wird die wässrige Fermentationsbrühe, vor oder nach Abtrennung der Mikroorganismen und sonstiger unlöslicher Bestandteile (Biomasse), mit einer starken Säure wie beispielsweise Schwefelsäure auf einen pH-Wert unterhalb 2 angesäuert, so dass die basische Aminosäure als Dikation vorliegt. Die angesäuerte wässrige Brühe wird dann über einen stark sauren Kationentauscher, dessen Säuregruppen in der Salzform vorliegen, z.B. als Natrium- oder Ammoniumsalze, geleitet, wodurch das Dikation der basischen Aminosäure auf dem Ionenaustauscherharz adsorbiert wird. Danach wird der so mit der basischen Aminosäure beladene Kationenaustauscher zur Entfernung von Verunreinigungen üblicherweise mit Wasser gewaschen. Anschließend wird die basische Aminosäure durch Behandlung mit einer verdünnten wässrigen Base, beispielsweise Natronlauge, Ammoniakwasser oder ein wässrigen Ammoniumpuffer, eluiert, wobei gleichzeitig die Salzform des Kationenaustauschers regeneriert wird. Aus dem so gewonnenen Eluat kann die basische Aminosäure, gegebenenfalls nach Ansäuern des Eluats, in üblicher Weise isoliert werden, z.B. durch Kristallisation.

Naturgemäß weist die beim Beladen des Kationenaustauschers mit dem Dikation der basischen Aminosäure ablaufende Flüssigkeit (Ablauf bzw. Effluent) eine hohe Salzfracht auf und kann daher als "High Density Waste Water" (HDWW) bezeichnet werden. Auch bei dem sich gegebenenfalls anschließenden Waschschritt fallen große Abwassermengen mit Salzfracht an ("Low Density Waste Water " (LDWW)). Diese Abwässer müssen zur Verringerung der Salzfracht einer aufwändigen Abwasserbehandlung unterzogen werden. Alternativ kann man die salzhaltigen Abwässer entwässern und das dabei anfallende Konzentrat entsorgen oder einer anderen Verwendung zuführen. Beide Maßnahmen sind jedoch mit einem zusätzlichen apparativen Aufwand und einem hohen Energieaufwand verbunden und tragen daher zu einem nicht unbeträchtlichen Anteil zu den Kosten der fermentativen Aminosäureherstellung bei. Es hat daher nicht an Versuchen gefehlt, die Salzfracht und die Abwassermenge, welche bei einer Aufarbeitung von basische Aminosäure enthaltenden Fermentationsbrühen mittels Kationenaustauscher anfallen, zu reduzieren.

Hsiao et al., Biotechnology and Bioengineering Vol. 49 (1996) S. 341 - 347, schlagen zur Verringerung der Abwassermenge die Rückführung der am Kationenaustauscher anfallenden salzhaltigen Effluente in das Fermentationsmedium vor. Neben der Gefahr, dass hierdurch Fermentationsinhibitoren, welche im Metabolismus der Mikroorganismen üblicherweise als Nebenprodukte gebildet werden, im Fermentationsmedium akkumulieren, hat sich gezeigt, dass hierbei die Bindungskapazität des Kationenaustauschers herabgesetzt wird, sodass die durch die verringerte Abwassermenge erreichte Kostenersparnis durch die Kosten für eine größere Kationenaustauscheranordnung aufgezehrt werden.

I. Lee et al., Enzyme and Microbiol. Technol. 30 (2002) S. 798-803 schlagen zur Verringerung der Salzfracht bei der Aufarbeitung Lysin-haltiger Fermentationsbrühen den Einsatz der Ionenausschlusschromatographie anstelle des üblicherweise eingesetzten Kationenaustauschers vor. Hierzu wird zunächst der Feststoffanteil der Fermentationsbrühe mittels Mikrofiltration entfernt. Die so erhaltene wässrige, Lysin-haltige Brühe wird auf den isoelektrischen Punkt (pH 9,74) eingestellt und dann durch einen Kationentauscher geleitet. Da die ionischen Bestandteile der Brühe nicht absorbiert werden, finden sich diese im Effluenten wieder. Die Aminosäure wird anschließend mit Wasser eluiert. Es hat sich jedoch gezeigt, dass eine hohe Wiedergewinnungsrate an L-Lysin von mehr als 90% nur dann erreicht wird, wenn sowohl die Menge an Lysin-haltigem Feed als auch die Durchflussrate gering sind. Trotz der geringeren Salzfracht ist daher diese Aufarbeitungsform nicht wirtschaftlich.

Die US 4,714,767 wiederum beschreibt ein mehrstufiges Verfahren zur Abtrennung basischer Aminosäuren aus einer wässrigen Brühe mittels einer Anordnung von mehreren, in Serie geschalteten Kationenaustauschersäulen, bei dem man den letzten Teil des beim Beladen der ersten Säule anfallenden Effluenten in den Beladungsvorgang einer späteren Abtrennung zurückführt. Auch wird vorgeschlagen, den letzten Teil des Eluats der ersten Säule in den Elutionsvorgang einer späteren Abtrennung zurückzuführen. Auf diese Weise wird die Wassermenge reduziert, nicht jedoch die Salzfracht.

EP 1,106,602 A1 beschreibt die Abtrennung von Aminosäuren aus Verunreinigungen enthaltenden, wässrigen Lösungen der Aminosäuren durch "Simulated Moving Bed" Chromatographie an einem stark sauren Kationenaustauscher. Gemäß einer bevorzugten Ausführungsform setzt man hierzu eine Vorrichtung ein, die eine oder mehrere seriell verschalteten Chromatographiesäulen mit einem stark sauren Kationenaustauscher umfasst und die in sequentieller Anordnung einen ersten Desorptionsport, einen Extraktport, einen Feedport, einen Raffinatport und einen zweiten Desorptionsport umfasst. Zur Abtrennung der Aminosäure bringt man simultan (i) durch den Feedport eine aminosäurehaltige Feedlösung und (ii) durch den ersten Desorptionsport einen basischen Eluenten mit dem Kationenaustauscher-Material in Kontakt, zieht (iii) durch den zweiten Desorptionsport gegebenenfalls eine wässrige Lösung ab und (iv) zieht durch den Extraktport eine Lösung ab, welche die Aminosäure enthält und eine im Vergleich zur Feedlösung geringere Menge an Verunreinigungen aufweist. Vorteile hinsichtlich einer Verringerung der Salzfracht und der Abwassermenge sind nicht ersichtlich.

Der vorliegenden Erfindung liegt daher die Aufgabe zugrunde, ein Verfahren zur Gewinnung basischer Aminosäuren aus der Fermentationsbrühe eines die basische Aminosäure produzierenden Mikroorganismenstamms bereitzustellen, das die hier geschilderten Nachteile des Standes der Technik überwindet.

Es wurde überraschenderweise gefunden, dass diese Aufgabe durch ein Verfahren gelöst wird, bei dem man zunächst die Hauptmenge des Mikroorganismus aus der Fermentationsbrühe abtrennt (Schritt a) und anschließend die basische Aminosäure aus der so erhaltenen wässrigen Brühe durch sukzessives Beladen einer ein- oder mehrstufigen, in der Regel seriellen Anordnung eines stark sauren Kationenaustauschers in seiner Salzform mit der Brühe und Eluieren der basischen Aminosäure abtrennt (Schritt b), wenn die wässrige Brühe vor dem Beladen einen pH-Wert im Bereich von 4,0 bis 7,5, insbesondere 4,5 bis 7,2 und speziell 4,6 bis 7 aufweist und wenigstens die erste Stufe der Kationenaustauscheranordnung mit einer wässrigen Säure in einer Weise vorbehandelt wird, dass am Ende der Vorbehandlung der pH-Wert im Effluenten des vorbehandelten Kationenaustauschers im Bereich von 4,5 bis 7,0 liegt.

Dementsprechend betrifft die vorliegende Erfindung das hier und in den Ansprüchen dargelegte Verfahren zur Gewinnung einer basischen Aminosäure aus der Fermentationsbrühe eines die basische Aminosäure produzierenden Mikroorganismenstamms. Das erfindungsgemäße Verfahren ist mit einer Reihe von Vorteilen verbunden: zum einen ist die beim erfindungsgemäßen Verfahren anfallende Salzmenge und damit die Salzfracht des Abwassers geringer als in den Verfahren des Standes der Technik, bei denen Kationenaustauscher zur Abtrennung und Gewinnung der basischen Aminosäure aus der Fermentationsbrühe genutzt werden. Zudem erreicht man hohe Ausbeuten von in der Regel größer 95% an basischer Aminosäure auch bei hohen Beladungs- und Durchflussraten am Kationenaustauscher. Aufgrund der gewählten pH-Werte der Brühe tritt keine nennenswerte Ausfällung von Verunreinigungen auf, welche den Kationenaustauscher blockieren können und daher den Bedarf an Waschwasser erhöhen würden.

Erfindungsgemäß trennt man in einem ersten Schritt die Hauptmenge der in der Fermentationsbrühe enthaltenen Mikroorganismen und gegebenenfalls sonstige, vorhandene Feststoffe aus der Fermentationsbrühe ab. Eine vollständige Abtrennung dieser Bestandteile ist prinzipiell nicht erforderlich. Üblicherweise wird man jedoch wenigstens 70 % und insbesondere wenigstens 80 % der in der Fermentationsbrühe enthaltenen Feststoffe, einschließlich Mikroorganismen, in Schritt a) abtrennen. Vorzugsweise enthält die dabei erhaltene wässrige Brühe weniger als 1 Vol.-% und insbesondere nicht mehr als 0,6 Vol.-% an Zellmaterialien.

Das Abtrennen der Mikroorganismen und sonstiger fester Bestandteile kann in für die Abtrennung von Mikroorganismen üblicher Weise durch Filtration, einschließlich Kuchen- und Tiefenfiltration, Cross-Flow-Filtration, durch Membrantrennverfahren wie Ultra- und Mikrofiltration, durch Zentrifugation und Dekantieren, durch Einsatz von Hydrozyklonen, einer Kombination der aufgeführten Verfahren oder in sonstiger Weise erfolgen.

Es hat sich bewährt, vor der Abtrennung die Mikroorganismen in der Fermentationsbrühe zu inaktivieren (Sterilisieren der Fermentationsbrühe), beispielsweise durch übliche Pasteurisierungsverfahren, z. B. durch Eintrag von Wärme und/oder Heißdampf. Hierzu können übliche Wärmetauscher, beispielsweise Rohrbündelwärmetauscher oder Plattenwärmetauscher eingesetzt werden.

Die nach dem Abtrennen der Hauptmenge an Mikroorganismen und gegebenenfalls sonstiger Feststoffe erhaltene Brühe weist üblicherweise einen Gehalt an basischer Aminosäure von 4 bis 30 Gew.-%, häufig 8 bis 20 Gew.-% und insbesondere 10 bis 15 Gew.-% auf. Der pH-Wert liegt häufig im Bereich von 5 bis 7,5 und insbesondere im Bereich von 6 bis 7. Daher kann auf eine Einstellung des pH-Wertes der wässrigen Brühe, z.B. auf ein Ansäuern wie im Stand der Technik, verzichtet werden. Grundsätzlich kann jedoch eine Einstellung des pH-Wertes in den oben angegebene Bereichen vorgenommen werden, beispielsweise durch Zugabe geringer Mengen einer Säure, vorzugsweise einer Säure mit einem pKs-Wert < 4,5 wie Ameisensäure, Essigsäure oder Schwefelsäure. In dieser Ausführungsform weist daher die Brühe vorzugsweise einen pH-Wert im Bereich von 4,0 bis 6, insbesondere 4,5 bis 5,5 auf.

Aus der so erhaltenen wässrigen Brühe wird anschließend in Schritt b) die basische Aminosäure mit Hilfe einer Kationenaustauscheranordnung abgetrennt. Die Abtrennung in Schritt b) umfasst erfindungsgemäß wenigstens einen Vorbehandlungsschritt, einen sich anschließenden Beladungsschritt, bei der die basische Aminosäure auf dem stark sauren Ionenaustauscher adsorbiert wird, und wenigstens einen Elutionsschritt, durch den die basische Aminosäure vom Ionenaustauscher desorbiert wird. Diese Schritte können in der angegebenen Reihenfolge mehrfach wiederholt werden und es können zwischen den Schritten Waschschritte mit Wasser durchgeführt werden.

Die im erfindungsgemäßen Verfahren eingesetzte Kationenaustauscheranordnung umfasst einen oder vorzugsweise mehrere, z.B. 2, 3, 4 oder mehrere, bis zu 50, in Serie (seriell) geschaltete Stufen, üblicherweise in Form von Ionenaustauschersäulen, die als stationäre Phase einen oder mehrere stark saure Kationenaustauscher enthalten.

Als stark saure Kationenaustauscher kommen grundsätzlich alle Ionenaustauschermaterialien, z.B. organische Ionenaustauscherharze oder anorganische Ionenaustauscher, in Betracht, die stark saure Gruppen, in der Regel Sulfonatgruppen, aufweisen. Häufig handelt es sich um partikelförmige, mäßig oder stark vernetzte organische Polymere, häufig auf der Basis von Polystyrol, die an der Oberfläche der Polymerpartikel eine Vielzahl stark saure Gruppen aufweisen. Die durchschnittliche Anzahl der sauren Gruppen liegt üblicherweise im Bereich von 1 bis 4 meq/ml Ionentauscherharz. Die mittlere Teilchengröße der Ionentauscherpartikel liegt typischerweise im Bereich von 0,1 bis 1 mm, wobei größere als auch kleinere Teilchengrößen je nach Abmessung der Ionentauscheranordnung geeignet sein können. Die Polymerteilchen können beispielsweise gel-artig sein oder eine makroporöse Struktur aufweisen.

Derartige Ionentauscher sind bekannt und werden z.T. kommerziell für die Aufreinigung von Aminosäuren angeboten, beispielsweise unter den Handelsbezeichnungen Lewatit® K oder Lewatit® S der Fa. Bayer Aktiengesellschaft, z.B. Lewatit® K 2629, Lewatit® S110, Lewatit® S110H, Lewatit® S1467, Lewatit® S1468, Lewatit® S2568, Lewatit® S2568H, Amberjet®, Amberlyst® oder Amberlite® der Fa. Rohm & Haas, z.B. Amberjet® 1200, Amberjet® 1500, Amberlite® 200, Amberlite® 250, Amberlite® IRV120, Amberlite® IR 120, Amberlite® IR 200C, Amberlite® CG 6000, Amberlyst® 119 Wet, Dowex® der Firma Dow Chemicals, z.B. Dowex® 50X1-100, Dowex® 50X2-100, Dowex® 50X2-200, Dowex® 50X2-400, Dowex® 50X4-100, Dowex® 50X4-200, Dowex® 50X4-400, Dowex® 50X8-100, Dowex® 50X8-200, Dowex® 50X8-400, Dowex® 40X1-100, Dowex® 40X1-100, Dowex® 40X1-100, Dowex® HCR-S, Dowex® HCR-W2, Dowex® MSC-1, Dowex® 650C, Dowex® G26, Dowex® 88, Dowex® Monosphere 88, Dowex® Monosphere 99K/320, Dowex® Monosphere 99K/350, Dowex® Monosphere 99Ca/320, Dowex® Marathon C, Dowex® 032, Dowex® 406, Dowex® 437, Dowex® C500ES, Dowex® XUS 43518, Dowex® XUS 40406.00, Diaion® der Fa. Mitsubishi Corp., z.B. Diaion® SK1B, Diaion® SK1BS, Diaion® SK104, Diaion® SK112, Diaion® SK116, Diaion® 1-3561, Diaion® 1-3565, Diaion® 1-3570, Diaion® 1-3573, Diaion® 1-3577, Diaion® 1-3581, Duolite® D 5427, Duolite® D 5552 (organisch basierte Kationenaustauscher), weiterhin Adsorbosphere® SCX, Bakerbond® SCX, Partisil® SCX, Spherisorb® SCX, Supelcosil® LC3-SCX, Ultralsil® SCX und Zorbax® 300 SCX (Silica-basierter Kationenaustauscher).

Die Kationenaustauscheranordnung kann absatzweise betrieben werden und weist dann ein oder mehrere, z.B. 2, 3 oder 4, in Serie (seriell) geschaltete, stationäre Ionentauscherfestbetten auf. Sie kann auch kontinuierlich betrieben werden und weist dann in der Regel 5 bis 50 und insbesondere 15 bis 40 Ionentauscherbetten auf, die z.B. Bestandteil einer "True Moving Bed"-Anordnung (siehe K. Tekeuchi J. Chem. Eng. Japan 11 (1978 S. 216-220), einer "Continuos Circulating Annular"-Anordnung (siehe J.P. Martin, Discuss. Farraday Soc. 1949, S. 7) oder einer "Simulated Moving Bed"-Anordnung, wie beispielsweise in US 2,985,589 und WO 01/72689 sowie von G.J. Rossiter et al. Proceedings of AIChE Conference, Los Angeles, CA, Nov. 1991 oder H.J. Van Walsem et al. J. Biochtechnol. 59 (1997) S 127-123 beschrieben, sein können.

Vor dem erfindungsgemäßen Vorbehandlung des Kationenaustauschers mit der wässrigen Säure liegt der Kationenaustauscher in seiner Salzform vor, d.h. die stark sauren Gruppen des Kationenaustauschers liegen in deprotonierter Form vor und koordinieren zur Ladungsneutralität eine entsprechende Anzahl an Kationen. In der Regel handelt es sich bei den Kationen um Alkalimetallkationen, insbesondere um Natriumionen oder besonders bevorzugt um Ammoniumionen ($NH_4^+$).

Durch die erfindungsgemäße Vorbehandlung mit der schwachen wässrigen Säure wird ein Teil dieser Kationen eluiert und eine äquivalente Anzahl der sauren Gruppen reprotoniert, so dass auf dem Kationenaustauscher sowohl saure als auch basische Gruppen vorliegen. Man nimmt an, dass hierdurch die Bindungsfähigkeit der Ionenaustauscher für die basische Aminosäure erhöht und damit die Kapazität der Ionenaustauscheranordnung erhöht wird. Der Vorgang der Eluierung/Reprotonierung kann über den pH-Wert der während der Vorbehandlung am Kationenaustauscher ablaufenden wässrigen Flüssigkeit kontrolliert werden. Erfindungsgemäß ist der gewünschte Vorbehandlungsgrad erreicht, wenn der pH-Wert der ablaufenden Flüssigkeit (bestimmt bei 25°C) im Bereich von 4,5 bis 7 und insbesondere im Bereich von 5 bis 6,6 liegt.

Zur Vorbehandlung können grundsätzlich verdünnte Lösungen aller bekannten wässrigen Säuren eingesetzt werden. Es versteht sich von selber, dass diese Säuren vorzugsweise so gewählt sind, dass sie die Gewinnung der basischen Aminosäure aus dem Eluat nicht stören. Sofern es sich bei der Säure um eine von der zu isolierenden basischen Aminosäure verschiedene Säure handelt, werden solche Säuren bevorzugt, die nicht oder in sehr geringem Ausmaß vom Kationenaustauscher adsorbiert werden. Geeignet sind sowohl anorganische Säuren wie Schwefelsäure, Salzsäure, Salpetersäure und Phosphorsäure, organische Carbonsäuren mit vorzugsweise 1 bis 4 C-Atomen wie Ameisensäure, Essigsäure, Propionsäure, Polycarbonsäuren mit in der Regel 2 bis 4 Carboxylgruppen und gegebenenfalls Hydroxylgruppen wie Maleinsäure, Fumarsäure, Bernsteinsäure, Adipinsäure, Zitronensäure und dergleichen, Hydroxycarbonsäuren wie Milchsäure und Glykolsäure, und Sulfonsäuren, sowie die zu isolierenden Aminosäuren in protonierter Form, z.B. die Mono- und Dikationen der vorgenannten basischen Aminosäuren. Bevorzugte Säuren sind zum einen Säuren mit einem pKs-Wert < 4, insbesondere Ameisensäure und Schwefelsäure und andererseits die zu isolierende basische Aminosäure in Form ihres Monokations.

Die Konzentration an Säure in der wässrigen Lösung liegt üblicherweise im Bereich von 1 bis 85 g/l, insbesondere im Bereich von 2 bis 40 g/l. Wenn es sich um eine Säure handelt, die von einer basischen Aminosäure verschieden ist, beträgt die zur Erreichung des gewünschten Effekts erforderliche Menge an Säure üblicherweise 0,01 bis 0,2 mol/L Kationenaustauscher, insbesondere 0,02 bis 0,1 mol/L Kationenaustauscher. Sofern es sich bei der zur Vorbehandlung eingesetzten Säure um eine basische Aminosäure, z.B. die zu isolierende basische Aminosäure handelt, ist die Säuremenge vorzugsweise 0,3 bis 1,2 mol/L und insbesondere 0,5 bis 0,8 mol/L Kationenaustauscher. Die Menge an wässriger Säurelösung beträgt üblicherweise das 0,7 bis 10-fache des gesamten Bettvolumens der Kationenaustauscheranordnung.

Die spezifische Fließrate SV, d.h. das Verhältnis von durchschnittlicher Fließrate V (Volumengeschwindigkeit), mit der die wässrige Säure durch die Kationenaustauscheranordnung geleitet wird, zum Gesamtvolumen des Kationenaustauschers in der Kationenaustauscheranordnung (Bettvolumen BV), ist von untergeordneter Bedeutung und liegt typischerweise im Bereich von 0,1 bis 5 h$^{-1}$. Die Temperatur, bei der die Behandlung erfolgt liegt typischerweise im Bereich von 10 bis 80°C, vorzugsweise im Bereich von 20 bis 70°C und insbesondere im Bereich von 30 bis 60°C. Die Behandlung kann sowohl aufsteigend, d.h. die wässrige Säurelösung wird von unten nach oben durch die Säule(n) der Kationenaustauscheranordnung geleitet, als auch absteigend erfolgen, d.h. die wässrige Säurelösung wird von oben nach unten durch die Säule(n) der Kationenaustauscheranordnung geleitet.

Anschließend belädt man den so vorbehandelten Kationenaustauscher mit der basische Aminosäure, indem man die von der Biomasse befreite wässrige Brühe durch die Kationenaustauscheranordnung leitet. Die Beladung kann sowohl absteigend als auch aufsteigend erfolgen, wobei ersteres bevorzugt ist. Die Beladung erfolgt vorzugsweise mit einer spezifischen Fließrate im Bereich von 0,1 h$^{-1}$ bis 2 h$^{-1}$. Die Beladung erfolgt vorzugsweise bei einer Temperatur im Bereich von 20 bis 70°C und insbesondere im Bereich von 30 bis 60°C. Die Menge an wässriger Brühe wird üblicherweise so gewählt, dass wenigstens 60 % und insbesondere wenigstens 65% der in der wässrigen Brühe enthaltenen basischen Aminosäure adsorbiert werden. Die Menge an wässriger Brühe beträgt in der Regel die 0,5- bis 2-fache Menge des Bettvolumens. Je nach Adsorptionsgrad enthält der am Ausgang der Kationenaustauscheranordnung Effluent noch die basische Aminosäure, so dass der Effluent, ggf. nach Einstellung des pH-Werts, zur Vorbehandlung gemäß Ausführungsform A oder B eingesetzt werden kann. Üblicherweise ist jedoch eine pH-Wert-Einstellung nicht erforderlich.

Dem Beladungsvorgang kann sich ein Waschschritt anschließen. Hierzu wird Wasser, z.B. Prozesswasser, durch die Kationenaustauscheranordnung geleitet. Die Menge an Waschwasser beträgt auf dieser Stufe üblicherweise das 0,05- bis 0,3-fache des Bettvolumens. Die dabei anfallenden Waschwässer enthalten üblicherweise Verunreinigungen und werden verworfen. Sie können auch geringe Mengen der basischen Aminosäure enthalten und können dann mit dem beim Beladen anfallenden Effluenten vereinigt werden.

Dem Beladungs- bzw. dem gegebenenfalls durchgeführten Waschschritt schließt sich die Elution der basischen Aminosäure an. Hierzu leitet man eine wässrige Lösung einer Base (Eluent) durch die Kationenaustauscheranordnung. Hierdurch wird die basische Aminosäure desorbiert und eluiert und der Kationenaustauscher regeneriert, d.h. die sauren Gruppen des Kationenaustauschers werden wieder in die Salzform überführt. Die Basenkonzentration in dem Eluenten liegt üblicherweise im Bereich von 1 bis 10 Gew.-% und insbesondere im Bereich von 2 bis 8 Gew.-%. Geeignete Basen sind beispielsweise Ammoniak, Alkalimetallhydroxide und -carbonate, wobei Natronlauge und insbesondere Ammoniak bevorzugt werden. Die Menge an wässriger Base beträgt in der Regel die 0,5- bis 5-fache Menge des Bettvolumens. Bezüglich der Temperaturen und Fließrate gilt das für das Beladen gesagt. Die Elution kann sowohl aufsteigend als auch absteigend durchgeführt werden. Die Elution wird vorzugsweise in der gleichen Richtung wie die Beladung durchgeführt.

Der Elution kann sich ein weiterer Waschschritt anschließen, um gegebenenfalls vorhandene Verunreinigungen zu entfernen. Hierzu wird Wasser durch die Kationenaustauscheranordnung geleitet. Die Menge an Waschwasser beträgt auf dieser Stufe üblicherweise das 0,2- bis 1,3-fache des Bettvolumens. Der beim Waschritt anfallende Effluent wird als Abwasser mit geringer Salzfracht einer üblichen Abwasserbehandlung oder einer sonstigen Aufarbeitung zugeführt.

Das bei der Elution anfallende Eluat wird zur Gewinnung der Aminosäure in üblicher Weise aufgearbeitet. In der Regel wird man hierzu das Eluat aufkonzentrieren, z.B. durch Entfernen des Wasser in einer üblichen Verdampferanordnung.

Auf diese Weise erhält man eine konzentrierte wässrige Lösung der basischen Aminosäure, aus der diese durch Fällung oder Kristallisation, z.B. nach Zusatz von Salzsäure als Hydrochlorid, isoliert werden kann. Verfahren hierzu sind dem Fachmann bekannt und in der Literatur umfassend beschrieben (z.B. Hermann T. Industrial Production of amino acids by coryneform bacteria, J. of Biotechnology, 104(2003), 155-172).

Das beim Aufkonzentrieren anfallende wässrige Kondensat kann verworfen oder in den Prozess zurückgeführt werden. Beispielsweise kann das Kondensat in den Elutionsschritt der basischen Aminosäure in einer nachfolgenden Aminosäureabtrennung zurückgeführt werden. Vorzugsweise wird man hierzu das Kondensat im Anschluss an die Elution mit der wässrigen Base durch die Kationenaustauscheranordnung leiten. Der dabei anfallende Ablauf enthält häufig noch geringen Mengen an basischer Aminosäure und wird üblicherweise in die Elution einer nachfolgenden Aminosäureabtrennung zurückgeführt.

Im folgenden wird die Erfindung anhand zweier bevorzugter Ausführungsformen näher erläutert.

In einer ersten bevorzugten Ausführungsform (Ausführungsform A) der Erfindung setzt man zur Vorbehandlung des Kationenaustauschers eine wässrige Lösung der zu isolierenden basischen Aminosäure ein, die einen pH-Wert im Bereich von 4,5 bis 7,5 aufweist. Vorzugsweise weist die wässrige Lösung eine Konzentration an basischer Aminosäure im Bereich von 1 bis 85 g/l und insbesondere 2 bis 40 g/l auf. Die zur Erreichung des gewünschten Effekts erforderliche Menge an basischer Aminosäure beträgt üblicherweise 0,3 bis1,2 mol/L und insbesondere 0,5 bis 0,8 mol/L Kationenaustauscher. Die Menge an wässriger Säurelösung beträgt vorzugsweise das 0,7- bis 10-fache des gesamten Bettvolumens der Kationenaustauscheranordnung.

Zweckmäßigerweise setzt man als wässrige Lösung der basischen Aminosäure wenigstens einen Teil des Ablaufs einer Beladung der Kationenaustauscheranordnung aus einer vorangegangenen Aminosäureabtrennung ein, welcher die nicht absorbierte basische Aminosäure enthält. Bei einer mehrstufigen Kationenaustauscheranordnung umfasst die Lösung vorzugsweise wenigstens einen Teil, z.B. wenigstens 50 % oder die Gesamtmenge des Ablaufs der ersten Stufe.

Es versteht sich von selbst, dass bei einer erstmaligen Durchführung des Verfahrens gemäß Ausführungsform A ein solcher Ablauf noch nicht vorliegt. Daher muss zuvor die Kationenaustauscheranordnung angefahren werden. Hierzu leitet man die nach Abtrennung der Biomasse erhaltene wässrige Brühe durch die Kationenaustauscheranordnung, wobei der Kationenaustauscher mit der basischen Aminosäure beladen wird. Der dabei anfallende Effluent enthält üblicherweise noch nicht adsorbierte basische Aminosäure und weist einen pH-Wert in den oben angegebenen Grenzen auf. Der so anfallende Effluent kann dann zur Vorbehandlung der Kationenaustauscheranordnung eingesetzt werden.

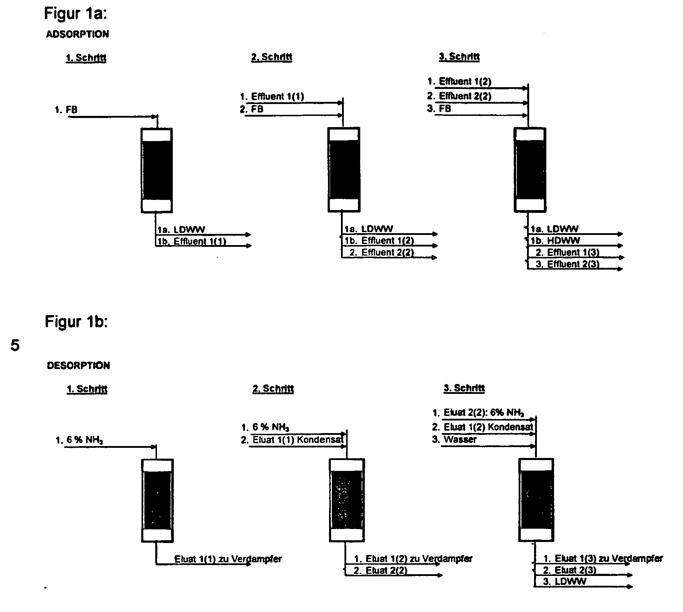

Die Figuren 1a und 1b zeigen die einzelnen Abläufe einer bevorzugten Ausgestaltung des Anfahrvorgangs. Zunächst wird die nach Abtrennung der Biomasse erhaltene Brühe FB durch die Kationenaustauscheranordnung (schematisch dargestellt) geleitet. Der erste Teil des Ablaufs wird als LDWW (Low Density Waster Water) bezeichnet und entspricht dem "freien Volumen" des Ionentauschers; der zweite Teil des Ablaufs wird als Effluent 1(1) aufgefangen. Anschließend wird die Kationenaustauscheranordnung erneut mit Effluent 1 (1) beaufschlagt. Der erste Teil des Ablaufs wird als LDWW abgezogen, der zweite Teil als Effluent 1(2)abgezogen. Im Anschluss erfolgt die Beladung mit Brühe FB, deren Ablauf als Effluent 2(2) abgezogen wird. Im nächsten Schritt wird die Kationenaustauscheranordnung in der Reihenfolge Effluent 1(2), Effluent 2(2), Brühe FB beaufschlagt. Bei Beaufschlagung mit dem Effluenten 1(2) fällt am Ablauf neben LDWW auch HDWW an. Hiernach erfolgt die Elution mit Ammoniakwasser (z.B. 6 % $NH_3$). Das hierbei anfallende Eluat 1, welches die basische Aminosäure enthält wird in einer Verdampferanordnung (nicht gezeigt) in Kondensat und Aminosäurekonzentrat getrennt. Es wird erneut mit Ammoniakwasser (z.B. 6 % $NH_3$) beaufschlagt (daraus resultiert am Ablauf wieder Eluat 1(2)) und anschließend mit dem Kondensat des Verdampfungsschrittes. Der dabei anfallende Ablauf wird als Eluat 2(Z) bezeichnet und zur Herstellung eines Ammoniakwassers verwendet, das im nächsten Schritt in den Ionentauscher geleitet wird. Das dabei anfallende Eluat 1(2) wird wieder in einer Verdampferanordnung in Kondensat und Aminosäurekonzentrat aufgetrennt und das anfallende Kondensat wieder auf den Ionentauscher geleitet (Abfluss: Eluat 2(3)). Anschließend wird zur Reinigung der Kationenaustauscheranordnung mit Wasser gewaschen und der Ablauf als LDWW der Entsorgung oder Aufarbeitung zugeführt.

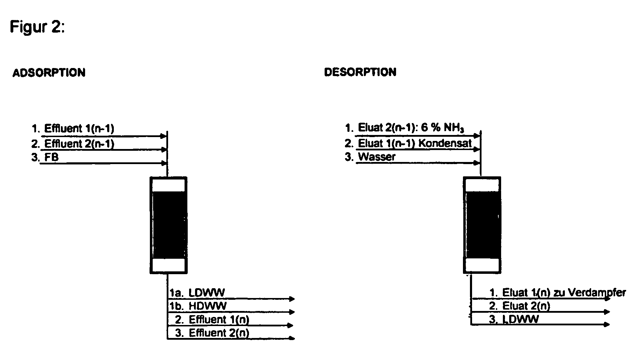

Figur 2 zeigt die einzelnen Abläufe einer bevorzugten Ausgestaltung der Ausführungsform A. Zunächst wird Effluent 1 des Anfahrvorgangs (bzw. Effluent 1(n-1) einer vorangegangenen Durchführung der Ausführungsform A) durch die Kationenaustauscheranordnung geleitet und der anfallende Effluent wird als LDWW und anschließend HDWW einer Entsorgung oder Aufarbeitung zugeführt. Anschließend wird Effluent 2 des Anfahrvorgangs (bzw. Effluent 2(n-1) einer vorangegangenen Durchführung der Ausführungsform A) durch die Kationenaustauscheranordnung geleitet. Der Ablauf wird als Effluent 1(n) aufgefangen und an der dem Effluenten 1(n+1) entsprechenden Stelle der Aminosäureabtrennung in einer Folgestufe zugeführt. Danach wird die nach Abtrennung der Biomasse aus der Fermentationsbrühe erhaltene wässrige Brühe FB durch die Kationenaustauscheranordnung geleitet. Der Ablauf wird als Effluent 2(n) aufgefangen und an der dem Effluenten 2(n+1) entsprechenden Stelle der Aminosäurenabtrennung in einer Folgestufe zugeführt. Danach erfolgt die Elution mit dem aus dem Anfahrvorgang bzw. aus der Vorstufe resultierendem Eluat 2(n-1), welches zu 6%-Ammoniakwasser angesetzt wurde. Hieraus resultiert Eluat 1(n) welches in einer Verdampferanordnung (nicht gezeigt) in Kondensat und Aminosäurekonzentrat getrennt wird. Das Kondensat wird der Folgestufe zugeführt. Als zweiter Elutionsschritt wird das Kondensat des Anfahrvorgangs oder der Vorstufe, Eluat 1(n-1), auf den Ionentauscher gegeben und der Ablauf als Eluat 2(n) aufgefangen und zu 6%igem Ammoniakwasser für die Folgestufe angesetzt. Anschließend wird zur Reinigung der Kationenaustauscheranordnung mit Wasser gewaschen und der Ablauf als LDWW der Entsorgung oder Aufarbeitung zugeführt.

In einer zweiten bevorzugten Ausführungsform B der Erfindung setzt man zur Vorbehandlung des Kationenaustauschers eine verdünnte wässrige Lösung einer Säure ein, deren $pK_S$-Wert nicht mehr als 4 beträgt. Bevorzugte Säuren sind Ameisensäure Phosphorsäure, Salzsäure und insbesondere Schwefelsäure sowie Gemische dieser Säuren.

Vorzugsweise weist die verdünnte wässrige Säurelösung eine Säurekonzentration im Bereich von 1 bis 20 g/l auf. Die zur Erreichung des gewünschten Effekts erforderliche Menge an Säure beträgt üblicherweise 0,01 bis 0,1 mol pro Liter Kationenaustauscher. Die Menge an wässriger Säurelösung beträgt vorzugsweise das 0,7- bis 2-fache des gesamten Bettvolumens der Kationenaustauscheranordnung.

In einer bevorzugten Ausgestaltung der Ausführungsform B wird im Anschluss an die Behandlung mit der verdünnten wässrigen Säurelösung eine Behandlung mit einer verdünnten wässrigen Lösung der basischen Aminosäure durchführt, worin wenigstens 90 % der basischen Aminosäure in einfach protonierter Form vorliegen. Diese Lösung weist üblicherweise einen pH-Wert im Bereich von 4,5 bis 7 auf. Vorzugsweise weist die wässrige Lösung eine Konzentration an basischer Aminosäure im Bereich von 8 bis 70 g/l und insbesondere 8 bis 50 g/l auf. Die Menge an wässriger Lösung der basischen Aminosäure beträgt vorzugsweise das 0,1 bis 1-fache des gesamten Bettvolumens der Kationenaustauscheranordnung.

In einer weiteren bevorzugten Ausgestaltung der Ausführungsform B wird zunächst nur mit einem Teil der verdünnten wässrigen Lösung der Säure mit $pK_S \leq 4$ eine Behandlung durchgeführt und dann wird eine Behandlung mit einer verdünnten wässrigen Lösung der basischen Aminosäure durchgeführt, worin wenigstens 90 % der basischen Aminosäure in einfach protonierter Form vorliegen. Bezüglich Konzentration und pH-Wert gilt das zuvor gesagte analog. Die Menge an wässriger Lösung der basischen Aminosäure beträgt vorzugsweise das 0,1 bis 1-fache des gesamten Bettvolumens der Kationenaustauscheranordnung. Zweckmäßigerweise setzt man auch hier als wässrige Lösung der basischen Aminosäure wenigstens einen Teil des Ablaufs einer Beladung der Kationenaustauscheranordnung aus einer vorangegangenen Aminosäureabtrennung ein, welcher die nicht absorbierte basische Aminosäure enthält. Im Anschluss an die Behandlung mit der wässrigen Lösung der basischen Aminosäure folgt dann in der Regel eine Behandlung mit der Restmenge der verdünnten wässrigen Lösung Säure mit einem $pK_S \leq 4$.

Die Effluenten, die bei der Behandlung der Kationenaustauscheranordnung der Ausführungsform B anfallen, werden in der Regel der Abwasserbehandlung zugeführt. Bezüglich der Waschwässer und der Rückführung von Eluenten und Kondensaten gilt das zuvor für die Ausführungsform A gesagte analog.

Das erfindungsgemäße Verfahren ist grundsätzlich für die Isolierung aller basischen Aminosäuren, insbesondere natürlicher Aminosäuren wie Lysin, Ornithin, Histidin oder Arginin anwendbar und wird insbesondere zur Isolierung von fermentativ hergestelltem L-Lysin eingesetzt.

Die Art des Fermentationsprozesses sowie der zur Herstellung der Aminosäure eingesetzte Mikroorganismenstamm spielen für das erfindungsgemäße Verfahren keine Rolle, so dass das erfindungsgemäße Verfahren zur Isolierung der basischen Aminosäure aus beliebigen Fermentationsbrühen geeignet ist.

In der Regel handelt es sich um Verfahren, bei denen man einen Mikroorganismenstamm, der die gewünschte basische Aminosäure produziert, in einem Fermentationsmedium kultiviert, das als Substrat mindestens eine Kohlenstoffquelle, z.B. Melasse und/oder Rohzucker, und eine Stickstoffquelle, z.B. Ammoniak oder Ammoniumsalze wie Ammoniumsulfat, sowie gegebenenfalls Mineralstoffe und Spurenelemente enthält. Diese Substratbestandteile können als solche oder in Form einer komplexen Mischung, z.B. als Corn-Steep-Liquor, eingesetzt werden.

Die Art des Mikroorganismenstamms richtet sich naturgemäß nach der Art der zu produzierenden Aminosäure. In der Regel handelt es sich um Stämme, welche die gewünschte basische Aminosäure überproduzieren. Im Falle des L-Lysins und des Histidins handelt es sich in der Regel um Stämme der Gattung *Corynebacterium* oder *Brevi bacterium,* z.B der *Spezies Corynebacterium glutamicum* oder *Brevibacterium lactofermentum,* im Falle des Arginins um Stämme der Spezies *Bacillus subtilis* oder *Brevibacterium flavum,* wobei jedoch in jüngerer Zeit auch Stämme anderer Gattungen eingesetzt werden.

In der Regel wird man die Fermentation so weit führen, dass der Gehalt an basischer Aminosäure in der Fermentationsbrühe im Bereich von 50 bis 200 g/L und insbesondere im Bereich von 80 bis 150 g/L liegt. Der Anteil an Biomasse, d.h. Mikroorganismen (als Biotrockenmasse) und sonstige unlösliche Bestandteile biologischen Ursprungs (z.B. Zellulosefasern aus der Glukosequelle), liegt üblicherweise im Bereich von 3 bis 7 Gew.-%. Daneben enthält die Fermentationsbrühe in der Regel noch Restmengen an Substrat, z.B. nicht verbrauchter Zucker sowie Nebenprodukte der Fermentation, z.B. saure oder neutrale Aminosäuren oder sonstige basische Aminsäuren, Peptide und dergleichen.

Die Fermentationsverfahren können kontinuierlich oder absatzweise als Batch- oder Fed-Batch-Verfahren durchgeführt werden. In der Regel handelt es sich um eine Fermentationsbrühe, die nach einem Fed-Batch-Verfahren hergestellt wurde, d.h. die Hauptmenge der Substrate wird der Mikroorganismen-haltigen Brühe im Verlauf der Fermentation zugeführt.

Derartige Verfahren und geeignete Mikroorganismenstämme sind dem Fachmann bekannt, z.B. aus dem eingangs zitierten Stand der Technik (siehe insbesondere Pfefferle et al. und Th. Herrmann, loc.cit) sowie aus WO 95/16042, WO 96/06180, WO 96/16042, WO 96/41042, WO 01/09306, EP-A 175309, EP-A 327945, EP-A 551614, EP-A 837134, US 4346170, US 5305576, US 6025165, US 6653454, DE 253199, GB 851396, GB 849370 und GB 1118719 (Herstellung von L-Lysin), EP-A 393708, GB 1098348, US 3668072, US 3574061, US 3532600, US 2988489, JP 2283290, JP 57016696 (L-Ornithin), US 3902967, US 4086137, GB 2084566 (Arginin) US 3875001 und US 3902966 (Histidin) bekannt.

Die Maßnahmen zur technischen Durchführung und Steuerung derartiger Fermenationen sind dem Fachmann geläufig und kann der einschlägigen Literatur, beispielsweise Storhas (s.o.) und J.E. Bailey et al. Biochemical Engineering Fundamentals, 2. ed. MacGraw-Hill 1986, Kapitel 9, entnommen werden.

Die Erfindung wird durch die folgenden Figuren sowie Beispiele und Vergleichsbeispiele erläutert, die bevorzugte Ausführungsformen des erfindungsgemäßen Verfahrens darstellen und nicht einschränkend zu verstehen sind.

Figuren 1a und 1b: Es werden schematisch die einzelnen Verfahrensschritte des Anfahrvorgangs der Ausführungsform A (Figur 1a: Beladung; Figur 1b: Desorption) gezeigt.

Figur 2: Es werden schematisch die einzelnen Verfahrensschritte der Ausführungsform A gezeigt.

Verwendete Abkürzungen:

| | |
|---|---|
| FB: | Fermentationsbrühe |
| HDWW: | Abwasser mit hoher Salzfracht |
| HDWW: | Abwasser mit geringer Salzfracht |
| ID: | Innendurchmesser |
| H: | Höhe |
| Lys-HCl: | L-Lysin-Monohydrochlorid |
| BV: | Bettvolumen (Volumen des Kationenaustauschers in der Anordnung) |
| SV: | spezifische Fließrate (Fließgeschwindigkeit in 1 $[m^3_{Feed}/(m^3_{Harz} h)]$, Einheit im folgenden abgekürzt als $[h^{-1}]$) |

Einsatzmaterialien:

Alle Versuche wurden mit einer L-Lysin-haltigen Fermentationsbrühe durchgeführt, die in an sich bekannter Weise durch Fermentation mit *C. glutamicum* hergestellt wurde. Die Fermentationsbrühe wies einen Gehalt an Lys-HCl von 110 bis 130 g/l und einen Gehalt an Biomasse (als Biotrockenmasse gerechnet) von 2,5 -3,5 Gew.-% auf. Der Salzgehalt lag zwischen 3 und 5 Gew.-%.

Kationenaustauscheranordnung

In Vergleichsbeispiel 1 und den Beispielen 1, 2 und 6 diente als Kationenaustauscheranordnung eine zylindrische Säule mit den Abmessungen 25 mm (ID) x 1200 mm (H), die mit 400 ml eines stark sauren Kationenaustauscherharzes beladen war. Als Kationenaustauscher diente ein sulfoniertes, vernetztes Polystyrol vom Geltyp mit einer mittleren Teilchengröße von etwa 0,6 mm (Lewatit® S 1468 der Firma Bayer Aktiengesellschaft) und einer Gesamtkapazität > 2 meq/ml. Die Kationenaustauscheranordnung wurde vor ihrer Benutzung mit 6 gew.-%igem wässrigem Ammoniak äquilibriert.

In den Beispielen 3 und 4 wurde eine Kationenaustauscheranordnung vom Typ "simulated moving bed" mit 30 zylindrischen Säulen mit den Abmessungen 33 mm (ID) x 1000 mm (H) (Typ "L 100 C" der Firma AST, USA) wobei die Kammern insgesamt mit 18 l eines stark sauren Kationenaustauschers (Typ Lewatit ® S1468 der Firma Bayer Aktiengesellschaft) gepackt waren.

In Beispiel 5 diente als Kationenaustauscheranordnung eine zylindrische Säule mit den Abmessungen 35 mm (ID) x 900 mm (H), die mit 600 ml eines stark sauren Kationenaustauscherharzes (Lewatit® S 1468) beladen war.

Die in Beispiel 7 eingesetzte Kationenaustauscheranordnung umfasste 9 in Serie geschaltete zylindrische Säulen mit den Abmessungen 35 mm (ID) x 900 mm (H), die mit jeweils 845 ml eines stark sauren Kationenaustauscherharzes ("Diaion SK 1 B" der Firma Mitsubishi, Japan) gepackt waren.

Alle Ionenaustauscheranordnungen wurden vor Ihrer erstmaligen Benutzung mit 6 gew.-%iger Ammoniaklösung äquilibriert.

Vergleichsbeispiel 1

Eine Fermentationsbrühe mit einem Lysingehalt von 12,5 Gew.-% und einem Biomasseanteil von 3 Gew.-% (als Biotrockenmasse) wird mit 0,7 g konzentrierter Schwefelsäure je g Lys-HCl auf einen pH-Wert von 1,5 angesäuert.

Die angesäuerte Fermentationsbrühe wurde dann bei einer Temperatur von 45°C durch die Kationenaustauscheranordnung geleitet. Der hierbei anfallende Effluent wurde aufgefangen und als HDWW entsorgt. Anschließend wurde die Kationenaustauscheranordnung mit Wasser gespült. Der hierbei anfallende Effluent wurde als LDWW entsorgt. Anschließend wurde L-Lysin mit 6 gew.-%igem wässrigem Ammoniak in einer Menge von 0,305 g Ammoniak/g Lys-HCl eluiert.

Beispiel 1

Die Fermentationsbrühe wurde erhitzt und mit Dampf bei einer Temperatur von 45 bis 70 °C sterilisiert. Anschließend entfernte man die Zellmasse durch eine Kombination von Zentrifugation und Dekantierung. Der wässrige Überstand wies eine Konzentration an Lysin von 12,5 Gew.-% auf. Der pH-Wert des Überstands lag bei 6,5.
Zunächst leitete man insgesamt 2040 ml wässrige Lysin-Lösung bestehend aus fünf verschiedenen Effluenten aus Vorstufen einer vorangegangenen Lysin-Isolierung mit einem Gehalt an Lysin von 0,36-8,45 w/v-% und einem pH-Wert von 4,5-7,5 bei einer Temperatur von 50 °C aufsteigend durch die Kationenaustauscheranordnung. Der pH-Wert des Ablaufs am Ende der Vorbehandlung lag bei 5,8. Anschließend leitete man 416 ml des Überstands der Zentrifugation aufsteigend bei einer Temperatur von 55 °C durch die so vorbehandelte Kationenaustauscheranordnung. Der hierbei anfallende Effluent wurde in einem der folgenden Beispiele zur Vorbehandlung eingesetzt. Im Anschluss daran wurde Lysin mittels 600 ml einer 6 gew.-%igen wässrigen Ammoniaklösung absteigend eluiert. Die so erhaltene, wässrige Lysin-Lösung kann in konventioneller Weise kristallisiert werden.

Beispiel 2

Die Durchführung des Beispiels 2 erfolgte analog zu Beispiel 1, wobei man im Unterschied zu Beispiel 1 zur Vorbehandlung 1248 ml wässrige Lysin-Lösung bestehend aus drei verschiedenen Effluenten aus Vorstufen einer vorangegangenen Lysin-Isolierung mit einem Gehalt an Lysin von 0,28-3,3 w/v-% und einem pH-Wert von 6,3-6,7 von oben nach unten durch die Kationenaustauscheranordnung leitete und die anschließende Beladung mit dem Überstand der Zentrifugation ebenfalls absteigend durchführt. Der pH-Wert des Ablaufs am Ende der Vorbehandlung lag bei 6,5.

Beispiel 3

Die Fermentationsbrühe wurde erhitzt und durch Einleiten von Dampf bei 45 bis 70 °C sterilisiert. Anschließend trennte man die Zellmasse durch Zentrifugation ab. Der so erhaltene Überstand hatte eine Lysinkonzentration von 12,5 Gew.-% und einen pH-Wert von 7,2.
Zur Vorbehandlung leitete man 55,1 l wässrige Lysin-Lösung mit einem Gehalt an Lysin von 0,36-8,45 w/v-% Gew.-% und einem pH-Wert von 4,5-7,5 bei einer Temperatur von 55 °C und einer Fliessrate von 5,5 l/h aufwärts durch die Kationenaustauscheranordnung. Der pH-Wert des Ablaufs am Ende der Vorbehandlung lag bei 5,8 Anschließend leitete man 9 l des bei der Zentrifugation erhaltenen Überstands bei einer Temperatur von 55 °C und einer Fließgeschwindigkeit von 5,5 l/h aufwärts durch die Kationenaustauscheranordnung. Anschließend eluierte man abwärts bei einer Temperatur von 55 °C und einer Fließrate von 5,5 l/h mit insgesamt 10,8 l einer 6 gew.-%igen, wässrigen Ammoniaklösung.

Beispiel 4

Beispiel 4 wurde analog Beispiel 3 durchgeführt, wobei man zur Vorbehandlung lediglich 33,7 l wässrige Lysin-Lösung bestehend aus drei verschiedenen Effluenten aus Vorstufen einer vorangegangenen Lysin-Isolierung mit einem Gehalt an Lysin von 0,28-3,3 w/v-% mit einem pH von 6,3-6,7 abwärts durch die Kationenaustauscheranordnung leitete. Der pH-Wert des Ablaufs am Ende der Vorbehandlung lag bei 6,5. Beladen wurde abwärts mit insgesamt 10,8 l des Überstands der Zentrifugation.

Beispiel 5

Die Fermentationsbrühe wurde durch Erwärmen und Einleiten von Dampf bei 45 bis 70 °C sterilisiert und anschließend in einen Zentrifugalabscheider überführt. Auf diese Weise erhielt man einen wässrigen, Lysin-haltigen Überstand mit einem Lysingehalt von 118,3 gll, einem pH-Wert von 6,7, der noch 0,5 Vol.-% an Zellmaterial enthielt.
Durch die Kationenaustauscheranordnung wurden bei einer Temperatur von 40 bis 50 °C mit einer Fließgeschwindigkeit von SV=1 h$^{-1}$ nacheinander 240 ml einer 0,44 gew.-%igen wässrigen Schwefelsäure, 500 ml einer 2,344 gew.-%igen, wässrigen Lysin-Lösung mit einem pH-Wert von 5 und anschließend 180 ml einer 0,44 gew.-%igen wässrigen Schwefelsäure geleitet. Der pH-Wert des Ablaufs lag bei 5,2. Danach leitete man unter Beibehaltung der Temperatur mit einer Fließgeschwindigkeit von SV=1 h⁻¹ 480 ml des wässrigen Lysin-haltigen Überstands und anschließend 120 ml Wasser auf den Ionentauscher. Der Effluent wurde abgetrennt und für eine Vorbehandlung in einem Folgeschritt bereitgestellt. Dann leitete man mit einer SV = 1 h⁻¹ bei 55 °C 650 ml einer 4 gew.-%igen wässrigen Ammoniaklösung und anschließend 720 ml Wasser durch die Kationenaustauscheranordnung.

Beispiel 6

Die Fermentationsbrühe wurde durch Erwärmen und Einleiten von Dampf bei 45 bis 70 °C sterilisiert und anschließend in einen Zentrifugalabscheider überführt. Auf diese Weise erhielt man einen wässrigen, Lysin-haltigen Überstand mit einem Lysingehalt von 121 g/l, einem pH-Wert von 6,7, der noch 0,5 Vol.-% an Zellmaterial enthielt.

Durch die Kationenaustauscheranordnung wurden bei einer Temperatur von 40 bis 50 °C mit einer spezifischen Fließrate SV = 1,0 nacheinander 160 ml einer 0,44 gew.-%igen wässrigen Schwefelsäure, 350 ml einer 1,23 gew.-%igen, wässrigen Lysin-Lösung mit einem pH-Wert von 5 und anschließend 120 ml einer 0,44 gew.-%igen wässrigen Schwefelsäure geleitet. Der pH-Wert des Ablaufs lag bei 5,2. Danach leitete man mit einer SV = 1 h⁻¹ unter Beibehaltung der Temperatur 320 ml des wässrigen Lysin-haltigen Überstands und anschließend 80 ml Wasser durch die Ionenaustauscheranordnung. Der Effluent wurde abgetrennt und für eine Vorbehandlung in einem Folgeschritt bereitgestellt. Dann leitete man mit einer SV = 1 h⁻¹ bei 55 °C 420 ml einer 4 gew.-%igen wässrigen Ammoniaklösung und anschließend 480 ml Wasser durch die Kationenaustauscheranordnung.

Beispiel 7

Die Fermentationsbrühe wurde durch Erwärmen und Einleiten von Dampf bei 45 bis 70 °C sterilisiert und anschließend in einen Zentrifugalabscheider überführt. Auf diese Weise erhielt man einen wässrigen, Lysin-haltigen Überstand mit einem Lysingehalt von 129,1 g/l, einem pH-Wert von 6,7, der noch 0,5 Vol.-% an Zellmaterial enthielt.

Durch die Kationenaustauscheranordnung wurden bei einer Temperatur von 40 bis 50 °C mit einer Fließgeschwindigkeit von SV =1 h⁻¹ nacheinander 2300 ml einer 0,66 gew.-%igen wässrigen Schwefelsäure, 4800 ml einer 0,882 gew.-%igen, wässrigen Lysin-Lösung mit einem pH-Wert von 5 und anschließend 1200 ml einer 0,66 gew.-%igen wässrigen Schwefelsäure geleitet. Der pH-Wert des Ablaufs lag bei 5,2. Danach leitete man unter Beibehaltung der Temperatur mit einer SV =1 h⁻¹ 6200 ml des wässrigen Lysin-haltigen Überstands und anschließend 1100 ml Wasser. Der Effluent wurde abgetrennt und für eine Vorbehandlung in einem Folgeschritt bereitgestellt. Dann leitete man mit einer SV = 1 h⁻¹ bei 55°C 6000 ml einer 4 gew.-%igen wässrigen Ammoniaklösung und anschließend 9100 ml Wasser durch die Kationenaustauscheranordnung.

Die Ergebnisse sind in den Tabellen 1a und 1 b zusammengestellt. Die Lysin-Ausbeute [%] wurde dabei wie folgt berechnet:

$$Lysin - Ausbeute = \frac{m_{Lys-HCL}(Eluat)}{m_{Lys-HCL}(adsorbiert)} \cdot 100\,[\%]$$

mit $m_{Lys-HCl}$ (Eluat): Lys-HCl Menge in Eluat [g]
   $m_{Lys-HCl}$ (adsorbiert): am Ionentauscher adsorbierte Lys-HCl Menge [g]

**Tabelle 1a:**

| Bsp. | Effluent für Vorbehandlung [L pro Liter Harz] | Lysin-Feed [1] [g Lys-HCl pro Liter Harz] | Schwefelsäure[2] [g/g Lys-HCl] | Ammoniak [g/g Lys-HCl] | HDWW [g/g Lys-HCl Eluat] | Lysin-Ausbeute [%] |
|---|---|---|---|---|---|---|
| V1 | - | 210 | 0,7 | 0,305 | 20,5 | 97,5 |
| 1 | 5 | 270 | - | 0,33 | 11,8 | 97,5 |
| 2 | 3 | 150 | - | 0,6 | 12,7 | 97,8 |

(fortgesetzt)

| Bsp. | Effluent für Vorbehandlung [L pro Liter Harz] | Lysin-Feed [1] [g Lys-HCl pro Liter Harz] | Schwefelsäure[2] [g/g Lys-HCl] | Ammoniak [g/g Lys-HCl] | HDWW [g/g Lys-HCl Eluat] | Lysin-Ausbeute [%] |
|---|---|---|---|---|---|---|
| 3 | 3,1 | 270 | - | 0,13 | 11,8 | 97,5 |
| 4 | 1,9 | 150 | - | 0,24 | 12,7 | 97,8 |

1) Gewichtsteile Lys-HCl pro Liter Kationenaustauscherharz
2) Schwefelsäure zum Ansäuern der Fermentationsbrühe

**Tabelle 1b:**

| Bsp. | Effluent für Vorbehandlung [L pro Liter Harz] | Lysin-Feed [g Lys-HCl pro Liter Harz] | Schwefelsäure [g/g Lys-HCl] | Ammoniak [g/g Lys-HCl] | HDWW [ml/(g-Lys-HCl Eluat)] | Ausbeute Lysin [%] |
|---|---|---|---|---|---|---|
| 5 | 0,83 | 105,8 | 0,029 | 0,4 | 13,2 | 96,8 |
| 6 | 0,88 | 107,6 | 0,029 | 0,39 | 12,2 | 97,3 |
| 7 | 0,63 | 110,9 | 0,027 | 0,28 | 10,8 | 97,5 |

**Patentansprüche**

1. Verfahren zur Gewinnung einer basischen Aminosäure aus der Fermentationsbrühe eines die basische Aminosäure produzierenden Mikroorganismenstamms, umfassend:

   a) Abtrennen der Mikroorganismen aus der Fermentationsbrühe und
   b) Abtrennen der basischen Aminosäure aus der in Schritt a) erhaltenen wässrigen Brühe durch sukzessives Beladen einer ein- oder mehrstufigen Anordnung eines stark sauren Kationenaustauschers in seiner Salzform mit der in Schritt a) erhaltenen Brühe und Eluieren der basischen Aminosäure,

   wobei die wässrige Brühe vor dem Beladen in Schritt b) einen pH-Wert im Bereich von 4 bis 7,5 aufweist und wenigstens die erste Stufe der Kationenaustauscheranordnung mit einer wässrigen Säure in einer Weise vorbehandelt wird, dass am Ende der Vorbehandlung der pH-Wert am Ablauf des vorbehandelten Kationenaustauschers im Bereich von 4,5 bis 7 liegt.

2. Verfahren nach Anspruch 1, wobei man den Kationenaustauscher mit einer wässrigen Lösung der basischen Aminosäure vorbehandelt, die einen pH-Wert im Bereich von 4,5 bis 7 aufweist.

3. Verfahren nach Anspruch 2, wobei die wässrige Lösung der basischen Aminosäure eine Konzentration an basischer Aminosäure im Bereich von 1 bis 85 g/l aufweist.

4. Verfahren nach Anspruch 2 oder 3, wobei man als wässrige Lösung der basischen Aminosäure wenigstens einen Teil des Ablaufs einer Beladung der Kationenaustauscheranordnung aus einer vorangegangenen Aminosäureabtrennung einsetzt.

5. Verfahren nach einem der Ansprüche 2 bis 4, wobei die zur Vorbehandlung eingesetzte Menge an Aminosäure 0,3 bis 1,2 mol pro Liter behandeltem Ionenaustauscher beträgt.

6. Verfahren nach Anspruch 1, wobei man den Kationenaustauscher mit einer verdünnten wässrigen Lösung einer Säure behandelt, deren $pK_s$-Wert nicht mehr als 4 beträgt.

7. Verfahren nach Anspruch 6, wobei die verdünnte wässrige Säurelösung eine Säurekonzentration im Bereich von 1 bis 20 g/l aufweist.

8. Verfahren nach einem der Ansprüche 6 oder 7, wobei die zur Behandlung eingesetzte Menge an Säureäquivalenten 0,01 bis 0,1 mol pro Liter behandeltem Ionenaustauscher beträgt.

9. Verfahren nach einem der Ansprüche 6 bis 8, wobei die Säure Schwefelsäure, Ameisensäure oder Salzsäure ist.

10. Verfahren nach einem der Ansprüche 6 bis 9, wobei man im Anschluss an die Behandlung mit verdünnter Säure eine Behandlung mit einer verdünnten wässrigen Lösung der basischen Aminosäure durchführt, worin wenigstens 90 % der basischen Aminosäure in einfach protonierter Form vorliegen.

11. Verfahren nach Anspruch 10, wobei man als verdünnte wässrige Lösung der basischen Aminosäure den Ablauf einer Beladung der ersten Stufe der Kationenaustauscheranordnung aus einer vorangegangenen Aminosäureabtrennung einsetzt.

12. Verfahren nach einem der vorhergehenden Ansprüche, wobei die sauren Gruppen des Ionenaustauschers vor dem Behandeln in Form von Ammonium- oder Natrium-Salzgruppen vorliegen.

13. Verfahren nach einem der vorhergehenden Ansprüche, wobei man in der Brühe vor dem Beladen durch Zugabe einer Säure einen pH-Wert im Bereich von 4,0 bis 6,0 und insbesondere im Bereich von 4,5 bis 5,5 einstellt.

14. Verfahren nach einem der vorhergehenden Ansprüche, wobei man die basische Aminosäure mit wässrigem Ammoniak oder Natronlauge eluiert.

15. Verfahren nach einem der vorhergehenden Ansprüche, wobei die basische Aminosäure Lysin ist.

16. Verfahren nach einem der vorhergehenden Ansprüche, wobei die Kationenaustauscheranordnung wenigstens 2 in Serie geschaltete Kationenaustauscherstufen umfasst.

17. Verfahren nach einem der vorhergehenden Ansprüche, wobei die Beladung des Kationentauschers bei Temperaturen im Bereich von 30 bis 60°C erfolgt.

18. Verfahren nach einem der vorhergehenden Ansprüche, umfassend zusätzlich die Isolierung der basischen Aminosäure durch Kristallisation aus dem Eluat.

19. Verfahren nach Anspruch 18, wobei man wobei man die basische Aminosäure mit wässrigem Ammoniak eluiert, das Eluat einengt und das dabei anfallende Kondensat zumindest teilweise in die Elution der basischen Aminosäure in einer nachfolgenden Aminosäureabtrennung zurückführt.

20. Verfahren nach einem der vorhergehenden Ansprüche, wobei Beladen und Eluieren abwärts erfolgt.

**Claims**

1. Method for obtaining a basic amino acid from the fermentation broth of a microorganism strain which produces the basic amino acid, comprising:

    a) separating the microorganisms from the fermentation broth and
    b) separating the basic amino acid from the aqueous broth obtained in step a) by a successive loading of a single or multi-step arrangement of a strongly acidic cation exchanger in its salt form with the broth obtained in step a) and eluting the basic amino acid,

    wherein the aqueous broth before loading in step b) has a pH in the range of 4 to 7.5 and wherein at least the first step of the cation exchanger arrangement is pretreated with an aqueous acid such that at the end of the pretreatment the pH at the exit point of the pretreated cation exchanger is in the range of 4.5 to 7.

2. Method according to claim 1, wherein the cation exchanger is pretreated with an aqueous solution of the basic amino acid, which has a pH in the range of 4.5 to 7.

3. Method according to claim 2, wherein the aqueous solution of the basic amino acid has a concentration of the basic amino acid in the range of 1 to 85 g/l.

4. Method according to claim 2 or 3, wherein at least a part of the outflow of a loading of the cation exchanger arrangement from a prior amino acid separation is used as aqueous solution of the basic amino acid.

5. Method according to any one of claims 2 to 4, wherein the amount of amino acid used for the pretreatment is 0.3 to 1.2 mol per liter of treated ion exchanger.

6. Method according to claim 1, wherein the cation exchanger is treated with a diluted aqueous solution of an acid whose $pK_s$ value does not exceed 4.

7. Method according to claim 6, wherein the diluted aqueous acid solution has an acid concentration in the range of 1 to 20 g/l.

8. Method according to claim 6 or 7, wherein the amount of acid equivalents for the treatment is 0.01 to 0.1 mol per liter of treated ion exchanger.

9. Method according to any one of claims 6 to 8, wherein the acid is sulfuric acid, formic acid or hydrochloric acid.

10. Method according to any one of claims 6 to 9, wherein subsequent to the treatment with diluted acid a treatment with a diluted aqueous solution of a basic amino acid is carried out, wherein at least 90 % of the basic amino acid is present in a single protonated form.

11. Method according to claim 10, wherein the outflow of the loading of the first step of the cation exchanger arrangement from a prior amino acid separation is used as diluted aqueous solution of the basic amino acid.

12. Method according to any of the preceding claims, wherein the acidic groups of the ion exchanger before the treatment are present in the form of ammonium or sodium salt groups.

13. Method according to any one of the preceding claims, wherein in the broth before the loading the pH is adjusted to 4.0 to 6.0, in particular to the range of 4.5 to 5.5 by the addition of an acid.

14. Method according to any one of the preceding claims, wherein the basic amino acid is eluted with aqueous ammonia or a sodium hydroxide solution.

15. Method according to any one of the preceding claims, wherein the basic amino acid is lysine.

16. Method according to any one of the preceding claims, wherein the cation exchanger arrangement comprises at least two serially connected cation exchanger steps.

17. Method according to any one of the preceding claims, wherein the loading of the cation exchanger is carried out at temperatures in the range of 30°C to 60°C.

18. Method according to any one of the preceding claims, comprising additionally the isolation of the basic amino acid by crystallization from the eluate.

19. Method according to claim 18, wherein the basic amino acid is eluted with aqueous ammonia, the eluate is concentrated and the thereby produced condensate is at least partially returned into the elution of the basic amino acid in a subsequent amino acid separation.

20. Method according to any one of the preceding claims, wherein the loading and elution is carried out downwards.

**Revendications**

1. Procédé permettant d'obtenir un acide aminé basique à partir du bouillon de fermentation d'une souche de microorganisme qui produit l'acide aminé basique, comprenant :

a) la séparation des micro-organismes à partir du bouillon de fermentation et

b) la séparation de l'acide aminé basique à partir du bouillon aqueux obtenu dans l'étape a) par un chargement successif d'un agencement en une seule étape ou en plusieurs étapes d'un échangeur de cations fortement acide sous la forme de son sel avec le bouillon obtenu dans l'étape a) et l'élution de l'acide aminé basique,

dans lequel le bouillon aqueux avant le chargement dans l'étape b) a un pH situé dans la plage de 4 à 7,5 et dans lequel au moins la première étape de l'agencement de l'échangeur de cations est prétraitée avec un acide aqueux de telle manière qu'à la fin du prétraitement, le pH au point de sortie de l'échangeur de cations prétraité se situe dans la plage de 4,5 à 7.

2. Procédé selon la revendication 1, dans lequel l'échangeur de cations est prétraité avec une solution aqueuse de l'acide aminé basique qui a un pH situé dans la plage de 4,5 à 7.

3. Procédé selon la revendication 2, dans lequel la solution aqueuse de l'acide aminé basique a une concentration de l'acide aminé basique située dans la plage de 1 à 85 g/l.

4. Procédé selon la revendication 2 ou 3, dans lequel au moins une partie de l'écoulement d'un chargement de l'agencement de l'échangeur de cations issu d'une séparation antérieure de l'acide aminé est utilisée en tant que solution aqueuse de l'acide aminé basique.

5. Procédé selon l'une quelconque des revendications 2 à 4, dans lequel la quantité d'acide aminé utilisée pour le prétraitement est de 0,3 à 1,2 mole par litre d'échangeur d'ions traité.

6. Procédé selon la revendication 1, dans lequel l'échangeur de cations est traité avec une solution aqueuse diluée d'un acide dont la valeur de $pK_S$ ne dépasse pas 4.

7. Procédé selon la revendication 6, dans lequel la solution d'acide aqueuse diluée a une concentration d'acide située dans la plage de 1 à 20 g/l.

8. Procédé selon la revendication 6 ou 7, dans lequel la quantité d'équivalents d'acide pour le traitement est de 0,01 à 0,1 mole par litre d'échangeur d'ions traité.

9. Procédé selon l'une quelconque des revendications 6 à 8, dans lequel l'acide est l'acide sulfurique, l'acide formique ou l'acide chlorhydrique.

10. Procédé selon l'une quelconque des revendications 6 à 9, dans lequel, suite au traitement avec l'acide dilué, un traitement avec une solution aqueuse diluée d'un acide aminé basique est réalisé, dans lequel au moins 90 % de l'acide aminé basique est présent sous une seule forme protonée.

11. Procédé selon la revendication 10, dans lequel l'écoulement du chargement de la première étape de l'agencement de l'échangeur de cations issu d'une séparation antérieure de l'acide aminé est utilisé en tant que solution aqueuse diluée de l'acide aminé basique.

12. Procédé selon l'une quelconque des revendications précédentes, dans lequel les groupes acides de l'échangeur d'ions avant le traitement sont présents sous la forme de groupes de sel d'ammonium ou de sodium.

13. Procédé selon l'une quelconque des revendications précédentes, dans lequel, dans le bouillon avant le chargement, le pH est ajusté à une valeur de 4,0 à 6,0, en particulier dans la plage de 4,5 à 5,5 par l'addition d'un acide.

14. Procédé selon l'une quelconque des revendications précédentes, dans lequel l'acide aminé basique est élué avec une solution aqueuse d'ammoniac ou d'hydroxyde de sodium.

15. Procédé selon l'une quelconque des revendications précédentes, dans lequel l'acide aminé basique est la lysine.

16. Procédé selon l'une quelconque des revendications précédentes, dans lequel l'agencement de l'échangeur de cations comprend au moins deux étapes d'échangeur de cations reliées en série.

17. Procédé selon l'une quelconque des revendications précédentes, dans lequel le chargement de l'échangeur de cations est réalisé à des températures situées dans la plage de 30 °C à 60 °C.

18. Procédé selon l'une quelconque des revendications précédentes, comprenant en outre l'isolement de l'acide aminé basique par cristallisation à partir de l'éluat.

19. Procédé selon la revendication 18, dans lequel l'acide aminé basique est élué avec de l'ammoniac aqueux, l'éluat est concentré et le condensat ainsi produit est au moins partiellement retourné dans l'élution de l'acide aminé basique dans une séparation ultérieure de l'acide aminé.

20. Procédé selon l'une quelconque des revendications précédentes, dans lequel le chargement et l'élution sont réalisés vers le bas.

## Figur 1a:

**ADSORPTION**

<u>1. Schritt</u>

1. FB

<u>2. Schritt</u>

1. Effluent 1(1)
2. FB

<u>3. Schritt</u>

1. Effluent 1(2)
2. Effluent 2(2)
3. FB

1a. LDWW
1b. Effluent 1(1)

1a. LDWW
1b. Effluent 1(2)
2. Effluent 2(2)

1a. LDWW
1b. HDWW
2. Effluent 1(3)
3. Effluent 2(3)

## Figur 1b:

**DESORPTION**

<u>1. Schritt</u>

1. 6 % NH$_3$

<u>2. Schritt</u>

1. 6 % NH$_3$
2. Eluat 1(1) Kondensat

<u>3. Schritt</u>

1. Eluat 2(2): 6% NH$_3$
2. Eluat 1(2) Kondensat
3. Wasser

Eluat 1(1) zu Verdampfer

1. Eluat 1(2) zu Verdampfer
2. Eluat 2(2)

1. Eluat 1(3) zu Verdampfer
2. Eluat 2(3)
3. LDWW

EP 1 874 945 B1

Figur 2:

ADSORPTION                              DESORPTION

1. Effluent 1(n-1)                      1. Eluat 2(n-1): 6 % $NH_3$
2. Effluent 2(n-1)                      2. Eluat 1(n-1) Kondensat
3. FB                                   3. Wasser

1a. LDWW                                1. Eluat 1(n) zu Verdampfer
1b. HDWW                                2. Eluat 2(n)
2. Effluent 1(n)                        3. LDWW
3. Effluent 2(n)

17

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4714767 A
- EP 1106602 A1
- US 2985589 A
- WO 0172689 A
- WO 9516042 A
- WO 9606180 A
- WO 9616042 A
- WO 9641042 A
- WO 0109306 A
- EP 175309 A
- EP 327945 A
- EP 551614 A
- EP 837134 A
- US 4346170 A
- US 5305576 A
- US 6025165 A
- US 6653454 B
- DE 253199
- GB 851396 A
- GB 849370 A
- GB 1118719 A
- EP 393708 A
- GB 1098348 A
- US 3668072 A
- US 3574061 A
- US 3532600 A
- US 2988489 A
- JP 2283290 A
- JP 57016696 L
- US 3902967 A
- US 4086137 A
- GB 2084566 A
- US 3875001 A
- US 3902966 A

**In der Beschreibung aufgeführte Nicht-Patentliteratur**

- Amino acids. **Axel Kleemann et al.** Ullmann's Encyclopedia of Industrial Chemistry. Wiley-VCH, 1997
- **Th. Hermann.** *J. Biotechnol.,* 2003, vol. 104, 155-172
- **Pfefferle et al.** *Adv. Biochem. Eng./Biotechnology,* 2003, vol. 79, 59-112
- **Atkinson et al.** Biochemical Engineering and Biotechnology Handbook. Stockton Press, 1991
- **Hsiao et al.** *Biotechnology and Bioengineering,* 1996, vol. 49, 341-347
- **I. Lee et al.** *Enzyme and Microbiol. Technol.,* 2002, vol. 30, 798-803
- **K. Tekeuchi.** *J. Chem. Eng. Japan,* 1978, vol. 11, 216-220
- **J.P. Martin.** *Discuss. Farraday Soc.,* 1949, 7
- **G.J. Rossiter et al.** *Proceedings of AlChE Conference,* November 1991
- **H.J. Van Walsem et al.** *J. Biochtechnol.,* 1997, vol. 59, 127-123
- **Hermann T.** Industrial Production of amino acids by coryneform bacteria. *J. of Biotechnology,* 2003, vol. 104, 155-172
- **J.E. Bailey et al.** Biochemical Engineering Fundamentals. MacGraw-Hill, 1986